# NEW EUROPEAN PATENT SPECIFICATION

(11) **EP 2 482 847 B2**
(45) Date of publication and mention of the opposition decision: **14.07.2021**
(45) Mention of the grant of the patent: 19.03.2014
(21) Application number: 10769061.2
(22) Date of filing: 28.09.2010
(51) Int. Cl.: A61K 39/385, B01D 61/14

(54) **PURIFICATION OF BACTERIAL VESICLES**
REINIGUNG VON BAKTERIELLEN VESIKELN
PURIFICATION DE VESICLES BACTÉRIENNES

(30) Priority: 28.09.2009 GB 0917003
(43) Date of publication of application: 08.08.2012
(73) Proprietor: GlaxoSmithKline Biologicals SA, 1330 Rixensart (BE)
(72) Inventor: Dl CIOCCIO, Vito, 53100 Siena (IT); COLUCCI, Anna Maria, 53100 Siena (IT); SAUL, Allan, 53100 Siena (IT)
(74) Representative: Klaczynska, Sarah Elizabeth
(86) International application number: PCT/IB2010/002556
(87) International publication number: WO 2011/036562

(56) References cited:
- WO-A1-89/00846
- WO-A1-2005/004908

## Description

### TECHNICAL FIELD

This invention is in the field of purifying vesicles from Gram-negative bacteria.

### BACKGROUND ART

Gram-negative bacteria can spontaneously release outer membrane blebs during growth due to the turgour pressure of the cell envelope. The formation of such blebs can be facilitated by disruption of certain bacterial components *e.g.* references 1 and 2 disrupted the MltA enzyme of meningococcus to provide strains which release vesicles into the culture medium during growth, and references 2 and 3 disrupted the *E.coli* Tol-Pal system for the same purpose.

Outer membrane vesicles (OMVs) can also be produced by disruption of whole bacteria. Known OMV production methods include methods which use detergent treatment (*e.g.* with deoxycholate) [4 & 5], detergent-free methods [6], or sonication [7], *etc.*

Various methods have been used to purify these immunogenic vesicles (*i.e.* blebs and OMVs). For instance, reference 8 reports an ultrafiltration-based method.

Although effective, these methods are labour intensive and expensive, particularly because of the use of centrifugation. Thus the methods are not suitable for the production of low cost vaccines against diseases which are common in developing countries *e.g.* against shigellosis. Thus there is a need for a simpler and cheaper process for the purification of immunogenic bacterial vesicles.

### DISCLOSURE OF THE INVENTION

The invention relates to a two-stage size filtration process to purify immunogenic bacterial vesicles. A first step separates the vesicles from intact bacteria based on their different sizes, with the smaller vesicles passing into the filtrate (permeate). A second step then uses a finer filter to remove smaller contaminants (*e.g.* soluble proteins), with the vesicles remaining in the retentate. This two stage process is extremely simple to operate but gives immunogenic vesicles of high purity.

Thus the invention provides a process for purifying immunogenic bacterial vesicles, obtained by disruption of or blebbing from the outer membrane of bacteria, from a composition which includes both whole bacteria and the vesicles obtained therefrom, consisting of: (i) a first tangential flow filtration step in which the vesicles are separated from the bacteria based on their different sizes, with the vesicles passing into the filtrate; and (ii) a second tangential flow filtration step wherein soluble proteins in the filtrate are removed from the vesicles and the vesicles are retained in the retentate. The retained vesicles can be used as an immunogenic component in a vaccine.

Also disclosed is a vesicle-containing composition obtained or obtainable by this process.

The invention also provides a process for preparing a pharmaceutical composition, such as a vaccine, comprising steps: (a) purifying immunogenic bacterial vesicles by a process of the invention; and
(b) formulating the purified vesicles with a pharmaceutically acceptable carrier (*e.g.* a buffer) and/or with an immunological adjuvant and/or with one or more further immunogenic components.

The invention also provides a process for preparing a pharmaceutical composition, such as a vaccine, comprising a step of formulating vesicles purified by a process of the invention with a pharmaceutically acceptable carrier (*e.g.* a buffer) and/or with an immunological adjuvant and/or with one or more further immunogenic components.

Also disclosed is a vesicle-containing pharmaceutical composition obtained or obtainable by these processes.

### The vesicles

The invention can be used for purifying various types of proteoliposomic vesicles which retain outer membrane proteins from bacteria. These proteoliposomic vesicle can be obtained by disruption of or blebbling from the outer membrane of a bacterium to form vesicles therefrom that include protein components of the outer membrane. Thus the term includes OMVs, blebs, microvesicles (MVs [9]) and 'native OMVs' ('NOMVs' [10]).

Blebs, MVs and NOMVs are naturally-occurring membrane vesicles that form spontaneously during bacterial growth and are released into culture medium. MVs can be obtained by culturing bacteria such as *Neisseria* in broth culture medium, separating whole cells from the smaller MVs in the broth culture medium (*e.g.* by filtration or by low-speed centrifugation to pellet only the cells and not the smaller vesicles), and then collecting the MVs from the cell-depleted medium (*e.g.* by filtration, by differential precipitation or aggregation of MVs, by high-speed centrifugation to pellet the MVs). Strains for use in production of MVs can generally be selected on the basis of the amount of MVs produced in culture *e.g.* refs. 11 & 12 describe *Neisseria* with high MV production. Hyperblebbing strains are disclosed in reference 13. Disruption of the *mltA* gene [1,2] can also provide meningococcal strains which spontaneously release suitable vesicles during culture. Disruption of the Tol-Pal system can be used to provide *E.coli* and *Shigella* strains which spontaneously release suitable vesicles during culture.

OMVs are prepared artificially from bacteria, and may be prepared using detergent treatment (*e.g.* with deoxycholate or sarkosyl), or by non-detergent means (*e.g.* see reference 14). Techniques for forming OMVs include treating bacteria with a bile acid salt detergent (*e.g.* salts of lithocholic acid, chenodeoxycholic acid, ursodeoxycholic acid, deoxycholic acid, cholic acid, ursocholic acid, *etc.,* with sodium deoxycholate [15 & 16] being preferred for treating *Neisseria*) at a pH sufficiently high not to precipitate the detergent [17]. Other techniques may be performed substantially in the absence of detergent [14] using techniques such as sonication, homogenisation, microfluidisation, cavitation, osmotic shock, grinding, French press, blending, *etc.* Methods using no or low detergent can retain useful antigens such as NspA [14]. Thus a method may use an OMV extraction buffer with about 0.5% deoxycholate or lower *e.g.* about 0.2%, about 0.1%, <0.05% or zero.

A useful process for OMV preparation is described in reference 18 and involves ultrafiltration on crude OMVs, rather than instead of high speed centrifugation. The process may involve a step of ultracentrifugation after the ultrafiltration takes place.

If LOS is present in a vesicle it is possible to treat the vesicle so as to link its LOS and protein components ("intra-bleb" conjugation [19]).

Preferred vesicles for use with the invention are produced by a *Shigella* bacterium (*e.g.* a *S.sonnei*) which does not express a functional TolR protein.

### The bacterium

The invention can be used to purify vesicles from various Gram negative bacteria, such as species in any of genera *Escherichia, Shigella, Neisseria, Moraxella, Bordetella, Borrelia, Brucella, Chlamydia Haemophilus, Legionella, Pseudomonas, Yersinia, Helicobacter, Salmonella, Vibrio, etc.*

For example, the bacterium may be *Bordetella pertussis, Borrelia burgdorferi, Brucella melitensis, Brucella ovis, Chlamydia psittaci, Chlamydia trachomatis, Moraxella catarrhalis, Escherichia coli, Haemophilus influenzae* (including non-typeable stains), *Legionella pneumophila, Neisseria gonorrhoeae, Neisseria meningitidis, Neisseria lactamica, Pseudomonas aeruginosa, Yersinia enterocolitica, Helicobacter pylori, Salmonella enterica* (including serovar *typhi* and *typhimurium), Vibrio cholerae, etc.*

The invention is particularly suitable for preparing vesicles from *Shigella* (such as *S.dysenteriae, S.flexneri, S. boydii* or *S. sonnei*) and *E.coli* (including extraintestinal pathogenic strains).

The bacterium can be a wild-type bacterium but, more typically, it will have been modified *e.g.* to inactivate genes which lead to a toxic phenotype. For example, it is known to modify bacteria so that they do not express a native lipopolysaccharide (LPS), particularly for *E.coli,* meningococcus, *Shigella,* and the like. Various modifications of native LPS can be made *e.g.* these may disrupt the native lipid A structure, the oligosaccharide core, or the outer O antigen. Absence of O antigen in the LPS is useful, as is absence of hexa-acylated lipid A. Inactivation of enterotoxins is also known *e.g.* to prevent expression of Shiga toxin.

A preferred bacterium for use with the invention is a *S.sonnei* strain with a *ΔtolR* genotype, including a strain with a *ΔtolRΔgalU* genotype.

### The first filtration

The first tangential flow filtration step separates the vesicles from intact bacteria based on their different sizes, with the smaller vesicles passing into the filtrate (permeate).

The input for the first tangential flow filtration step can be the product of a vesicle forming method (*e.g.* an OMV preparation method from meningococci). Usually, though, the input will be the culture medium of a blebbing bacterium.

This step can be a typical sterile filtration *e.g.* using a 0.22µm filter. The bacteria are retained by the filter but the vesicles pass through into the filtrate.

In general, the pore size for the first filtration will be selected according to the size and characteristics of the bacteria which are to be removed. The goal of the first filtration step is to retain more than 90% (by number) of intact bacteria, ideally >95%, >97%, >98%, >99% or >99.5%, and a pore size can be selected accordingly. For some bacteria (*e.g.* those with large cells) the first filtration step may be filtration through a 0.8µm, 0.65µm or 0.45µm pore size membrane, but for other bacteria (*e.g.* those with small cells) the first filtration step may be thought a 0.22µm or 0.2µm pore size membrane. Various suitable membranes are commercially available.

The first filtration step is performed with a tangential flow (cross-flow) arrangement. This arrangement helps to avoid clogging which is typical for dead-ended filtration and minimises the need for extensive pre-filtering. Reduced pre-filtering means that a lower volume of liquid remains trapped in the filters. Tangential flow microfiltration cassettes were evaluated in references 20 & 21, and are commercially available *e.g.* the MaxCell™ range of hollow fiber cartridges with 0.2µm pore size, or the MidGee™ cartridges with 0.2µm pore size, or ProCell™ hollow fiber cartridges with 0.2µm pore size (all available from GE Healthcare).

Tangential flow filtration in the first step is ideally performed with diafiltration. This permits efficient removal of filtrate components and involves addition of fresh solvent (*e.g.*a buffer, such as PBS) during the first filtration step. Addition of the fresh solvent can maintain the overall volume if it occurs at the same rate as solvent removal through the tangential flow filter.

### The second filtration

The second tangential flow filtration step uses a finer filter than the first step. Whereas the vesicles passed into the filtrate in the first filtration step, in the second filtration step they remain in the retentate.

In general, the pore size of the second filtration will be selected according to the size and characteristics of the vesicles which are to be retained. Some small vesicles may pass through the filter, but the goal of the second filtration step is to retain more than 50% (by number) of vesicles, ideally >60%, >70%, >80%, >90%, >95% while removing soluble proteins which are to be removed. Ideally >90% of total protein in the retentate should be part of the vesicles, with >10% as soluble protein. Suitable filters are usually quoted in terms of pore size (*e.g.* a suitable filter can have a pore size of 0.1 µm) or molecular weight (*e.g.* a 300kDa, 500kDa, 750kDa or 1000kDa membrane can be used). Various suitable membranes are commercially available.

The second filtration step is performed with A tangential flow (cross-flow) arrangement. as discussed above, this arrangement helps to avoid clogging. Tangential flow microfiltration cassettes are commercially available *e.g.* the MaxCell™ range of hollow fiber cartridges with 0.1µm pore size, or the MidGee™ cartridges with 0.1 µm pore size, or Xampler™ laboratory cartridges with 0.1 µm pore size (all available from GE Healthcare).

Tangential flow filtration in the second step is ideally performed with diafiltration (see above).

Retentate from the second filtration step contains vesicles and these may be resuspended in any suitable medium (*e.g.* in a buffer or other pharmaceutically acceptable liquid) ready for formulation into a vaccine.

### Pharmaceutical compositions

The invention provides a process for preparing a pharmaceutical composition comprising (a) purifying vesicles by a process of the invention and (b) formulating with a pharmaceutically acceptable carrier. The invention also provides a process for preparing such a composition, comprising the step of admixing vesicles purified by a process of the invention with a pharmaceutically acceptable carrier.

The immunogenic composition may include a pharmaceutically acceptable carrier, which can be any substance that does not itself induce the production of antibodies harmful to the patient receiving the composition, and which can be administered without undue toxicity. Pharmaceutically acceptable carriers can include liquids such as water, saline, glycerol and ethanol. Auxiliary substances, such as wetting or emulsifying agents, pH buffering substances, and the like, can also be present in such vehicles. A thorough discussion of suitable carriers is available in ref. 22.

Bacteria can affect various areas of the body and so the compositions of the invention may be prepared in various forms. For example, the compositions may be prepared as injectables, either as liquid solutions or suspensions. Solid forms suitable for solution in, or suspension in, liquid vehicles prior to injection can also be prepared. The composition may be prepared for topical administration *e.g.* as an ointment, cream or powder. The composition be prepared for oral administration *e.g.* as a tablet or capsule, or as a syrup (optionally flavoured). The composition may be prepared for pulmonary administration *e.g.* as an inhaler, using a fine powder or a spray. The composition may be prepared as a suppository or pessary. The composition may be prepared for nasal, aural or ocular administration *e.g.* as drops.

A pharmaceutical carrier may include a temperature protective agent, and this component may be particularly useful in adjuvanted compositions (particularly those containing a mineral adjuvant, such as an aluminium salt). As described in reference 23, a liquid temperature protective agent may be added to an aqueous vaccine composition to lower its freezing point *e.g.* to reduce the freezing point to below 0°C. Thus the composition can be stored below 0°C, but above its freezing point, to inhibit thermal breakdown. The temperature protective agent also permits freezing of the composition while protecting mineral salt adjuvants against agglomeration or sedimentation after freezing and thawing, and may also protect the composition at elevated temperatures *e.g.* above 40°C. A starting aqueous vaccine and the liquid temperature protective agent may be mixed such that the liquid temperature protective agent forms from 1-80% by volume of the final mixture. Suitable temperature protective agents should be safe for human administration, readily miscible/soluble in water, and should not damage other components (*e.g.*. antigen and adjuvant) in the composition. Examples include glycerin, propylene glycol, and/or polyethylene glycol (PEG). Suitable PEGs may have an average molecular weight ranging from 200-20,000 Da. In a preferred embodiment, the polyethylene glycol can have an average molecular weight of about 300 Da ('PEG-300').

The composition is preferably sterile. It is preferably pyrogen-free. It is preferably buffered *e.g.* at between pH 6 and pH 8, generally around pH 7. Compositions of the invention may be isotonic with respect to humans.

Immunogenic compositions comprise an immunologically effective amount of immunogenic vesicles, as well as any other of other specified components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (*e.g.* non-human primate, primate, *etc.*), the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials.

Previous work with vesicle vaccines (*e.g.* for meningococcus) offers pharmaceutical, posological and formulation guidance for compositions of the invention. The concentration of vesicles in compositions of the invention will generally be between 10 and 500 µg/ml, preferably between 25 and 200µg/ml, and more preferably about 50µg/ml or about 100µg/ml (expressed in terms of total protein in the vesicles). A dosage volume of 0.5ml is typical for injection.

The composition may be administered in conjunction with other immunoregulatory agents.

Adjuvants which may be used in compositions of the invention include, but are not limited to:

### A. Mineral-containing compositions

Mineral containing compositions suitable for use as adjuvants in the invention include mineral salts, such as aluminium salts and calcium salts. The invention includes mineral salts such as hydroxides (*e.g.* oxyhydroxides), phosphates (*e.g.* hydroxyphosphates, orthophosphates), sulphates, *etc.* [*e.g.* see chapters 8 & 9 of ref. 27], or mixtures of different mineral compounds, with the compounds taking any suitable form (*e.g.* gel, crystalline, amorphous, *etc.*), and with adsorption being preferred. The mineral containing compositions may also be formulated as a particle of metal salt.

The adjuvants known as "aluminium hydroxide" are typically aluminium oxyhydroxide salts, which are usually at least partially crystalline. Aluminium oxyhydroxide, which can be represented by the formula AlO(OH), can be distinguished from other aluminium compounds, such as aluminium hydroxide Al(OH)₃, by infrared (IR) spectroscopy, in particular by the presence of an adsorption band at 1070cm⁻¹ and a strong shoulder at 3090-3100cm⁻¹ [chapter 9 of ref. 27]. The degree of crystallinity of an aluminium hydroxide adjuvant is reflected by the width of the diffraction band at half height (WHH), with poorly-crystalline particles showing greater line broadening due to smaller crystallite sizes. The surface area increases as WHH increases, and adjuvants with higher WHH values have been seen to have greater capacity for antigen adsorption. A fibrous morphology (*e.g*. as seen in transmission electron micrographs) is typical for aluminium hydroxide adjuvants. The pI of aluminium hydroxide adjuvants is typically about 11 *i.e.* the adjuvant itself has a positive surface charge at physiological pH. Adsorptive capacities of between 1.8-2.6 mg protein per mg Al⁺⁺⁺ at pH 7.4 have been reported for aluminium hydroxide adjuvants.

The adjuvants known as "aluminium phosphate" are typically aluminium hydroxyphosphates, often also containing a small amount of sulfate (*i.e.* aluminium hydroxyphosphate sulfate). They may be obtained by precipitation, and the reaction conditions and concentrations during precipitation influence the degree of substitution of phosphate for hydroxyl in the salt. Hydroxyphosphates generally have a PO₄/Al molar ratio between 0.3 and 1.2. Hydroxyphosphates can be distinguished from strict AlPO₄ by the presence of hydroxyl groups. For example, an IR spectrum band at 3164cm⁻¹ (*e.g.* at 200°C) indicates the presence of structural hydroxyls [ch. 9 of ref. 27].

The PO₄/Al³⁺ molar ratio of an aluminium phosphate adjuvant will generally be between 0.3 and 1.2, preferably between 0.8 and 1.2, and more preferably 0.95+0.1. The aluminium phosphate will generally be amorphous, particularly for hydroxyphosphate salts. A typical adjuvant is amorphous aluminium hydroxyphosphate with PO₄/Al molar ratio between 0.84 and 0.92, included at 0.6mg Al³⁺/ml. The aluminium phosphate will generally be particulate (*e.g.* plate-like morphology as seen in transmission electron micrographs). Typical diameters of the particles are in the range 0.5-20µm (*e.g.* about 5-10µm) after any antigen adsorption. Adsorptive capacities of between 0.7-1.5 mg protein per mg Al⁺⁺⁺ at pH 7.4 have been reported for aluminium phosphate adjuvants.

The point of zero charge (PZC) of aluminium phosphate is inversely related to the degree of substitution of phosphate for hydroxyl, and this degree of substitution can vary depending on reaction conditions and concentration of reactants used for preparing the salt by precipitation. PZC is also altered by changing the concentration of free phosphate ions in solution (more phosphate = more acidic PZC) or by adding a buffer such as a histidine buffer (makes PZC more basic). Aluminium phosphates used according to the invention will generally have a PZC of between 4.0 and 7.0, more preferably between 5.0 and 6.5 *e.g.* about 5.7.

Suspensions of aluminium salts used to prepare compositions of the invention may contain a buffer (*e.g.* a phosphate or a histidine or a Tris buffer), but this is not always necessary. The suspensions are preferably sterile and pyrogen-free. A suspension may include free aqueous phosphate ions *e.g.* present at a concentration between 1.0 and 20 mM, preferably between 5 and 15 mM, and more preferably about 10 mM. The suspensions may also comprise sodium chloride.

In one embodiment, an adjuvant component includes a mixture of both an aluminium hydroxide and an aluminium phosphate. In this case there may be more aluminium phosphate than hydroxide *e.g.* a weight ratio of at least 2:1 *e.g.* ≥5:1, ≥6:1, ≥7:1, ≥8:1, ≥9:1, *etc.*

The concentration of Al⁺⁺⁺ in a composition for administration to a patient is preferably less than 10mg/ml *e.g.* ≤5 mg/ml, ≤4 mg/ml, ≤3 mg/ml, ≤2 mg/ml, ≤1 mg/ml, *etc.* A preferred range is between 0.3 and 1mg/ml. A maximum of <0.85mg/dose is preferred.

### B. Oil Emulsions

Oil emulsion compositions suitable for use as adjuvants in the invention include squalene-water emulsions, such as MF59 [Chapter 10 of ref. 27; see also ref. 24] (5% Squalene, 0.5% Tween 80, and 0.5% Span 85, formulated into submicron particles using a microfluidizer). Complete Freund's adjuvant (CFA) and incomplete Freund's adjuvant (IFA) may also be used.

Various suitable oin-in-water emulsions are known, and they typically include at least one oil and at least one surfactant, with the oil(s) and surfactant(s) being biodegradable (metabolisable) and biocompatible. The oil droplets in the emulsion are generally less than 5µm in diameter, and advantageously the emulsion comprises oil droplets with a sub-micron diameter, with these small sizes being achieved with a microfluidiser to provide stable emulsions. Droplets with a size less than 220nm are preferred as they can be subjected to filter sterilization.

The invention can be used with oils such as those from an animal (such as fish) or vegetable source. Sources for vegetable oils include nuts, seeds and grains. Peanut oil, soybean oil, coconut oil, and olive oil, the most commonly available, exemplify the nut oils. Jojoba oil can be used *e.g.* obtained from the jojoba bean. Seed oils include safflower oil, cottonseed oil, sunflower seed oil, sesame seed oil and the like. In the grain group, corn oil is the most readily available, but the oil of other cereal grains such as wheat, oats, rye, rice, teff, triticale and the like may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolizable and may therefore be used in the practice of this invention. The procedures for separation, purification, saponification and other means necessary for obtaining pure oils from animal sources are well known in the art. Most fish contain metabolizable oils which may be readily recovered. For example, cod liver oil, shark liver oils, and whale oil such as spermaceti exemplify several of the fish oils which may be used herein. A number of branched chain oils are synthesized biochemically in 5-carbon isoprene units and are generally referred to as terpenoids. Shark liver oil contains a branched, unsaturated terpenoid known as squalene, 2,6,10,15,19,23-hexamethyl-2,6,10,14,18,22-tetracosahexaene. Other preferred oils are the tocopherols (see below). Oil in water emulsions comprising sqlauene are particularly preferred. Mixtures of oils can be used.

Surfactants can be classified by their 'HLB' (hydrophile/lipophile balance). Preferred surfactants of the invention have a HLB of at least 10, preferably at least 15, and more preferably at least 16. The invention can be used with surfactants including, but not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (Span 85) and sorbitan monolaurate. Preferred surfactants for including in the emulsion are Tween 80 (polyoxyethylene sorbitan monooleate), Span 85 (sorbitan trioleate), lecithin and Triton X-100. As mentioned above, detergents such as Tween 80 may contribute to the thermal stability seen in the examples below.

Mixtures of surfactants can be used *e.g.* Tween 80/Span 85 mixtures. A combination of a polyoxyethylene sorbitan ester such as polyoxyethylene sorbitan monooleate (Tween 80) and an octoxynol such as t-octylphenoxypolyethoxyethanol (Triton X-100) is also suitable. Another useful combination comprises laureth 9 plus a polyoxyethylene sorbitan ester and/or an octoxynol.

Preferred amounts of surfactants (% by weight) are: polyoxyethylene sorbitan esters (such as Tween 80) 0.01 to 1%, in particular about 0.1 %; octyl- or nonylphenoxy polyoxyethanols (such as Triton X-100, or other detergents in the Triton series) 0.001 to 0.1 %, in particular 0.005 to 0.02%; polyoxyethylene ethers (such as laureth 9) 0.1 to 20 %, preferably 0.1 to 10 % and in particular 0.1 to 1 % or about 0.5%.

Specific oil-in-water emulsion adjuvants useful with the invention include, but are not limited to:
- A submicron emulsion of squalene, Tween 80, and Span 85. The composition of the emulsion by volume can be about 5% squalene, about 0.5% polysorbate 80 and about 0.5% Span 85. In weight terms, these ratios become 4.3% squalene, 0.5% polysorbate 80 and 0.48% Span 85. This adjuvant is known as 'MF59' [24-26], as described in more detail in Chapter 10 of ref. 27 and chapter 12 of ref. 28. The MF59 emulsion advantageously includes citrate ions *e.g.* 10mM sodium citrate buffer.
- An emulsion comprising squalene, an α-tocopherol, and polysorbate 80. These emulsions may have from 2 to 10% squalene, from 2 to 10% tocopherol and from 0.3 to 3% Tween 80, and the weight ratio of squalene:tocopherol is preferably ≤1 (*e.g.* 0.90) as this provides a more stable emulsion. Squalene and Tween 80 may be present volume ratio of about 5:2, or at a weight ratio of about 11:5. One such emulsion can be made by dissolving Tween 80 in PBS to give a 2% solution, then mixing 90ml of this solution with a mixture of (5g of DL-α-tocopherol and 5ml squalene), then microfluidising the mixture. The resulting emulsion may have submicron oil droplets *e.g.* with an average diameter of between 100 and 250nm, preferably about 180nm.
- An emulsion of squalene, a tocopherol, and a Triton detergent (*e.g.* Triton X-100). The emulsion may also include a 3d-MPL (see below). The emulsion may contain a phosphate buffer.
- An emulsion comprising a polysorbate (*e.g.* polysorbate 80), a Triton detergent (*e.g.* Triton X-100) and a tocopherol (*e.g.* an α-tocopherol succinate). The emulsion may include these three components at a mass ratio of about 75:11:10 (*e.g.* 750µg/ml polysorbate 80, 110µg/ml Triton X-100 and 100µg/ml α-tocopherol succinate), and these concentrations should include any contribution of these components from antigens. The emulsion may also include squalene. The emulsion may also include a 3d-MPL (see below). The aqueous phase may contain a phosphate buffer.
- An emulsion of squalane, polysorbate 80 and poloxamer 401 ("Pluronic™ L121"). The emulsion can be formulated in phosphate buffered saline, pH 7.4. This emulsion is a useful delivery vehicle for muramyl dipeptides, and has been used with threonyl-MDP in the "SAF-1" adjuvant [29] (0.05-1% Thr-MDP, 5% squalane, 2.5% Pluronic L121 and 0.2% polysorbate 80). It can also be used without the Thr-MDP, as in the "AF" adjuvant [30] (5% squalane, 1.25% Pluronic L121 and 0.2% polysorbate 80). Microfluidisation is preferred.
- An emulsion comprising squalene, an aqueous solvent, a polyoxyethylene alkyl ether hydrophilic nonionic surfactant (*e.g.* polyoxyethylene (12) cetostearyl ether) and a hydrophobic nonionic surfactant (*e.g.* a sorbitan ester or mannide ester, such as sorbitan monoleate or 'Span 80'). The emulsion is preferably thermoreversible and/or has at least 90% of the oil droplets (by volume) with a size less than 200 nm [31]. The emulsion may also include one or more of: alditol; a cryoprotective agent (*e.g.* a sugar, such as dodecylmaltoside and/or sucrose); and/or an alkylpolyglycoside. Such emulsions may be lyophilized.
- An emulsion having from 0.5-50% of an oil, 0.1-10% of a phospholipid, and 0.05-5% of a non-ionic surfactant. As described in reference 32, preferred phospholipid components are phosphatidylcholine, phosphatidylethanolamine, phosphatidylserine, phosphatidylinositol, phosphatidylglycerol, phosphatidic acid, sphingomyelin and cardiolipin. Submicron droplet sizes are advantageous.
- A submicron oil-in-water emulsion of a non-metabolisable oil (such as light mineral oil) and at least one surfactant (such as lecithin, Tween 80 or Span 80). Additives may be included, such as QuilA saponin, cholesterol, a saponin-lipophile conjugate (such as GPI-0100, described in reference 33, produced by addition of aliphatic amine to desacylsaponin via the carboxyl group of glucuronic acid), dimethyidioctadecylammonium bromide and/or N,N-dioctadecyl-N,N-bis (2-hydroxyethyl)propanediamine.
- An emulsion comprising a mineral oil, a non-ionic lipophilic ethoxylated fatty alcohol, and a non-ionic hydrophilic surfactant (*e.g.* an ethoxylated fatty alcohol and/or polyoxyethylenepolyoxypropylene block copolymer) [34].
- An emulsion comprising a mineral oil, a non-ionic hydrophilic ethoxylated fatty alcohol, and a non-ionic lipophilic surfactant (*e.g.* an ethoxylated fatty alcohol and/or polyoxyethylenepolyoxypropylene block copolymer) [34].
- An emulsion in which a saponin (*e.g.* QuilA or QS21) and a sterol (*e.g.* a cholesterol) are associated as helical micelles [35].

Antigens and adjuvants in a composition will typically be in admixture at the time of delivery to a patient. The emulsions may be mixed with antigen during manufacture, or extemporaneously, at the time of delivery. Thus the adjuvant and antigen may be kept separately in a packaged or distributed vaccine, ready for final formulation at the time of use. The antigen will generally be in an aqueous form, such that the vaccine is finally prepared by mixing two liquids. The volume ratio of the two liquids for mixing can vary (*e.g*. between 5:1 and 1:5) but is generally about 1:1.

### C. Saponin formulations [chapter 22 of ref. 27]

Saponin formulations may also be used as adjuvants in the invention. Saponins are a heterogeneous group of sterol glycosides and triterpenoid glycosides that are found in the bark, leaves, stems, roots and even flowers of a wide range of plant species. Saponin from the bark of the *Quillaia saponaria* Molina tree have been widely studied as adjuvants. Saponin can also be commercially obtained from *Smilax ornata* (sarsaprilla), *Gypsophilla paniculata* (brides veil), and *Saponaria officianalis* (soap root). Saponin adjuvant formulations include purified formulations, such as QS21, as well as lipid formulations, such as ISCOMs. QS21 is marketed as Stimulon™.

Saponin compositions have been purified using HPLC and RP-HPLC. Specific purified fractions using these techniques have been identified, including QS7, QS17, QS18, QS21, QH-A, QH-B and QH-C. Preferably, the saponin is QS21. A method of production of QS21 is disclosed in ref. 36. Saponin formulations may also comprise a sterol, such as cholesterol [37].

Combinations of saponins and cholesterols can be used to form unique particles called immunostimulating complexs (ISCOMs; see chapter 23 of ref. 27; also refs 38 & 39). ISCOMs typically also include a phospholipid such as phosphatidylethanolamine or phosphatidylcholine. Any known saponin can be used in ISCOMs. Preferably, the ISCOM includes one or more of QuilA, QHA & QHC. Optionally, the ISCOMS may be devoid of additional detergent [40].

A review of the development of saponin based adjuvants can be found in refs. 41 & 42.

### D. Bacterial or microbial derivatives

Adjuvants suitable for use in the invention include bacterial or microbial derivatives such as non-toxic derivatives of enterobacterial lipopolysaccharide (LPS), Lipid A derivatives, immunostimulatory oligonucleotides and ADP-ribosylating toxins and detoxified derivatives thereof.

Non-toxic derivatives of LPS include monophosphoryl lipid A (MPL) and 3-O-deacylated MPL (3dMPL). 3dMPL is a mixture of 3 de-O-acylated monophosphoryl lipid A with 4, 5 or 6 acylated chains. A preferred "small particle" form of 3 De-O-acylated monophosphoryl lipid A is disclosed in ref. 43. Such "small particles" of 3dMPL are small enough to be sterile filtered through a 0.22µm membrane [43]. Other non-toxic LPS derivatives include monophosphoryl lipid A mimics, such as aminoalkyl glucosaminide phosphate derivatives *e.g.* RC-529 [44,45].

Lipid A derivatives include derivatives of lipid A from *Escherichia coli* such as OM-174. OM-174 is described for example in refs. 46 & 47.

Immunostimulatory oligonucleotides suitable for use as adjuvants in the invention include nucleotide sequences containing a CpG motif (a dinucleotide sequence containing an unmethylated cytosine linked by a phosphate bond to a guanosine). Double-stranded RNAs and oligonucleotides containing palindromic or poly(dG) sequences have also been shown to be immunostimulatory.

The CpG's can include nucleotide modifications/analogs such as phosphorothioate modifications and can be double-stranded or single-stranded. References 48, 49 and 50 disclose possible analog substitutions *e.g.* replacement of guanosine with 2'-deoxy-7-deazaguanosine. The adjuvant effect of CpG oligonucleotides is further discussed in refs. 51-56.

The CpG sequence may be directed to TLR9, such as the motif GTCGTT or TTCGTT [57]. The CpG sequence may be specific for inducing a Th1 immune response, such as a CpG-A ODN, or it may be more specific for inducing a B cell response, such a CpG-B ODN. CpG-A and CpG-B ODNs are discussed in refs. 58-60. Preferably, the CpG is a CpG-A ODN.

Preferably, the CpG oligonucleotide is constructed so that the 5' end is accessible for receptor recognition. Optionally, two CpG oligonucleotide sequences may be attached at their 3' ends to form "immunomers". See, for example, refs. 61-63.

A particularly useful adjuvant based around immunostimulatory oligonucleotides is known as IC-31™ [64-66]. Thus an adjuvant used with the invention may comprise a mixture of (i) an oligonucleotide (*e.g.* between 15-40 nucleotides) including at least one (and preferably multiple) CpI motifs (*i.e.* a cytosine linked to an inosine to form a dinucleotide), and (ii) a polycationic polymer, such as an oligopeptide (*e.g.* between 5-20 amino acids) including at least one (and preferably multiple) Lys-Arg-Lys tripeptide sequence(s). The oligonucleotide may be a deoxynucleotide comprising 26-mer sequence 5'-(IC)₁₃-3' (SEQ ID NO: 7). The polycationic polymer may be a peptide comprising 11-mer amino acid sequence KLKLLLLLKLK (SEQ ID NO: 6). This combination of SEQ ID NOs: 6 and 7 provides the IC-31™ adjuvant.

Bacterial ADP-ribosylating toxins and detoxified derivatives thereof may be used as adjuvants in the invention. Preferably, the protein is derived from *E. coli* (*E. coli* heat labile enterotoxin "LT"), cholera ("CT"), or pertussis ("PT"). The use of detoxified ADP-ribosylating toxins as mucosal adjuvants is described in ref. 67 and as parenteral adjuvants in ref. 68. The toxin or toxoid is preferably in the form of a holotoxin, comprising both A and B subunits. Preferably, the A subunit contains a detoxifying mutation; preferably the B subunit is not mutated. Preferably, the adjuvant is a detoxified LT mutant such as LT-K63, LT-R72, and LT-G192. The use of ADP-ribosylating toxins and detoxified derivatives thereof, particularly LT-K63 and LT-R72, as adjuvants can be found in refs. 69-76. A useful CT mutant is or CT-E29H [77]. Numerical reference for amino acid substitutions is preferably based on the alignments of the A and B subunits of ADP-ribosylating toxins set forth in ref. 78, specifically incorporated herein by reference in its entirety.

### E. Human immunomodulators

Human immunomodulators suitable for use as adjuvants in the invention include cytokines, such as interleukins (*e.g.* IL-1, IL-2, IL-4, IL-5, IL-6, IL-7, IL-12 [79], *etc.*) [80], interferons (*e.g.* interferon-γ), macrophage colony stimulating factor, and tumor necrosis factor. A preferred immunomodulator is IL-12.

### F. Bioadhesives and Mucoadhesives

Bioadhesives and mucoadhesives may also be used as adjuvants in the invention. Suitable bioadhesives include esterified hyaluronic acid microspheres [81] or mucoadhesives such as cross-linked derivatives of poly(acrylic acid), polyvinyl alcohol, polyvinyl pyrollidone, polysaccharides and carboxymethylcellulose. Chitosan and derivatives thereof may also be used as adjuvants in the invention [82].

### G. Microparticles

Microparticles may also be used as adjuvants in the invention. Microparticles (*i.e.* a particle of ∼100nm to ∼150µm in diameter, more preferably ∼200nm to ∼30µm in diameter, and most preferably ∼500nm to ∼10µm in diameter) formed from materials that are biodegradable and non-toxic (*e.g.* a poly(α-hydroxy acid), a polyhydroxybutyric acid, a polyorthoester, a polyanhydride, a polycaprolactone, *etc.*) with poly(lactide-co-glycolide) are preferred, optionally treated to have a negatively-charged surface (*e.g.* with SDS) or a positively-charged surface (*e.g.* with a cationic detergent, such as CTAB).

### H. Liposomes (Chapters 13 & 14 of ref. 27)

Examples of liposome formulations suitable for use as adjuvants are described in refs. 83-85.

### I. Imidazoquinolone Compounds.

Examples of imidazoquinolone compounds suitable for use adjuvants in the invention include Imiquamod and its homologues (*e.g.* "Resiquimod 3M"), described further in refs. 86 and 87.

The invention may also comprise combinations of aspects of one or more of the adjuvants identified above. For example, the following adjuvant compositions may be used in the invention: (1) a saponin and an oil-in-water emulsion [88]; (2) a saponin (*e.g.* QS21) + a non-toxic LPS derivative (*e.g.* 3dMPL) [89]; (3) a saponin (*e.g.* QS21) + a non-toxic LPS derivative (*e.g.* 3dMPL) + a cholesterol; (4) a saponin (*e.g.* QS21) + 3dMPL + IL-12 (optionally + a sterol) [90]; (5) combinations of 3dMPL with, for example, QS21 and/or oil-in-water emulsions [91]; (6) SAF, containing 10% squalane, 0.4% Tween 80™, 5% pluronic-block polymer L121, and thr-MDP, either microfluidized into a submicron emulsion or vortexed to generate a larger particle size emulsion. (7) Ribi™ adjuvant system (RAS), (Ribi Immunochem) containing 2% squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); and (8) one or more mineral salts (such as an aluminum salt) + a non-toxic derivative of LPS (such as 3dMPL).

Other substances that act as immunostimulating agents are disclosed in chapter 7 of ref. 27.

An aluminium hydroxide adjuvant is useful, and antigens are generally adsorbed to this salt. Oil-in-water emulsions comprising squalene, with submicron oil droplets, are also preferred, particularly in the elderly. Useful adjuvant combinations include combinations of Th1 and Th2 adjuvants such as CpG & an aluminium salt, or resiquimod & an aluminium salt. A combination of an aluminium salt and 3dMPL may be used.

### Immunisation

Compositions produced by the processes of the invention also find use in a method for raising an antibody response in a mammal, comprising administering an immunogenic composition of the invention to the mammal. The antibody response is preferably a protective antibody response.

The compositions also find use in a method for protecting a mammal against a bacterial infection and/or disease, comprising administering to the mammal an immunogenic composition of the invention.

The compositions may be used as medicaments (*e.g.* as immunogenic compositions or as vaccines).

The mammal is preferably a human. The human may be an adult or, preferably, a child. Where the vaccine is for prophylactic use, the human is preferably a child (*e.g.* a toddler or infant); where the vaccine is for therapeutic use, the human is preferably an adult. A vaccine intended for children may also be administered to adults *e.g.* to assess safety, dosage, immunogenicity, *etc.*

The uses and methods are particularly useful for preventing/treating diseases caused by *Shigella* including, but not limited to, shigellosis, Reiter's syndrome, and/or hemolytic uremic syndrome.

Efficacy of therapeutic treatment can be tested by monitoring bacterial infection after administration of the composition of the invention. Efficacy of prophylactic treatment can be tested by monitoring immune responses against immunogenic proteins in the vesicles or other antigens after administration of the composition. Immunogenicity of compositions can be determined by administering them to test subjects (*e.g.* children 12-16 months age) and then determining standard serological parameters. These immune responses will generally be determined around 4 weeks after administration of the composition, and compared to values determined before administration of the composition. Where more than one dose of the composition is administered, more than one post-administration determination may be made.

Compositions will generally be administered directly to a patient. Direct delivery may be accomplished by parenteral injection (*e.g.* subcutaneously, intraperitoneally, intravenously, intramuscularly, or to the interstitial space of a tissue), or by rectal, oral, vaginal, topical, transdermal, intranasal, ocular, aural, pulmonary or other mucosal administration. Intramuscular administration to the thigh or the upper arm is preferred. Injection may be via a needle (*e.g.* a hypodermic needle), but needle-free injection may alternatively be used. A typical intramuscular dose is about 0.5 ml.

Dosage treatment can be a single dose schedule or a multiple dose schedule. Multiple doses may be used in a primary immunisation schedule and/or in a booster immunisation schedule. A primary dose schedule may be followed by a booster dose schedule. Suitable timing between priming doses (*e.g.* between 4-16 weeks), and between priming and boosting, can be routinely determined.

### Culture methods

Also disclosed herein is a process for culturing a *Shigella* bacterium, comprising growing the bacteria under agitated and aerated conditions at 37°C and pH 7.1 with dissolved oxygen at 30% saturation.

### General

The term "comprising" encompasses "including" as well as "consisting" *e.g.* a composition "comprising" X may consist exclusively of X or may include something additional *e.g.* X + Y.

The term "about" in relation to a numerical value *x* is optional and means, for example, *x*±10%.

The word "substantially" does not exclude "completely" *e.g.* a composition which is "substantially free" from Y may be completely free from Y. Where necessary, the word "substantially" may be omitted from the definition of the invention.

References to a percentage sequence identity between two amino acid sequences means that, when aligned, that percentage of amino acids are the same in comparing the two sequences. This alignment and the percent homology or sequence identity can be determined using software programs known in the art, for example those described in section 7.7.18 of reference 92. A preferred alignment is determined by the Smith-Waterman homology search algorithm using an affine gap search with a gap open penalty of 12 and a gap extension penalty of 2, BLOSUM matrix of 62. The Smith-Waterman homology search algorithm is well known and is disclosed in reference 93.

"GI" numbering is used above. A GI number, or "GenInfo Identifier", is a series of digits assigned consecutively to each sequence record processed by NCBI when sequences are added to its databases. The GI number bears no resemblance to the accession number of the sequence record. When a sequence is updated (*e.g.* for correction, or to add more annotation or information) then it receives a new GI number. Thus the sequence associated with a given GI number is never changed.

Where the invention concerns an "epitope", this epitope may be a B-cell epitope and/or a T-cell epitope. Such epitopes can be identified empirically (*e.g*. using PEPSCAN [94,95] or similar methods), or they can be predicted (*e.g.* using the Jameson-Wolf antigenic index [96], matrix-based approaches [97], MAPITOPE [98], TEPITOPE [99,100], neural networks [101], OptiMer & EpiMer [102, 103], ADEPT [104], Tsites [105], hydrophilicity [106], antigenic index [107] or the methods disclosed in references 108-109, *etc.*). Epitopes are the parts of an antigen that are recognised by and bind to the antigen binding sites of antibodies or T-cell receptors, and they may also be referred to as "antigenic determinants".

### BRIEF DESCRIPTION OF DRAWINGS

Figure 1 shows blebs of the invention purified from culture.
Figure 2 shows SDS-PAGE analysis of samples taken (i) before the first filtration, (ii) after the first filtration, and (iii) after the second filtration. Each panel has three lanes showing, from left to right, total protein, vesicle protein and soluble protein.
Figure 3 shows a SEC trace of samples taken after the first and second filtration steps.

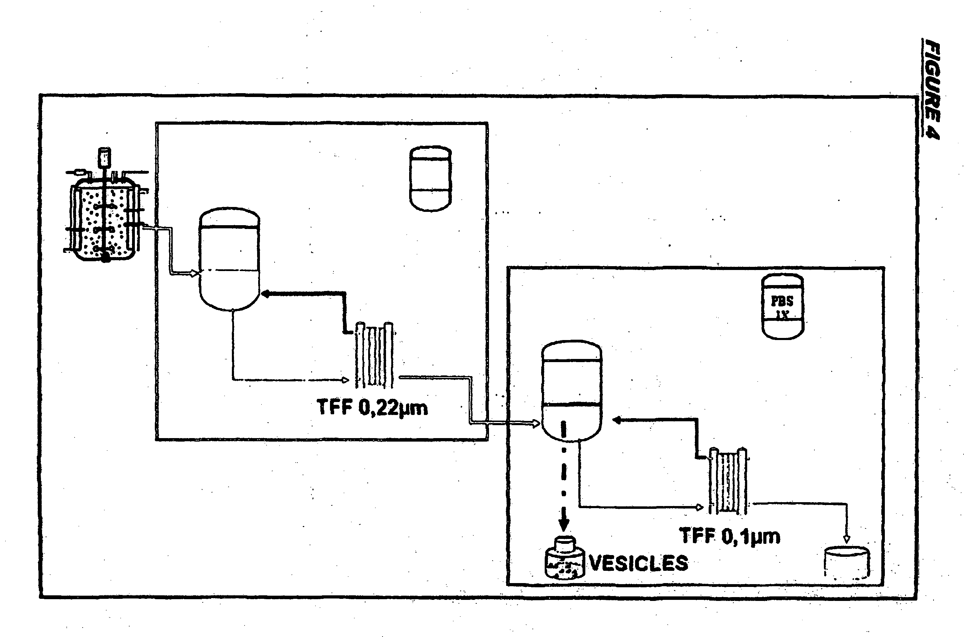

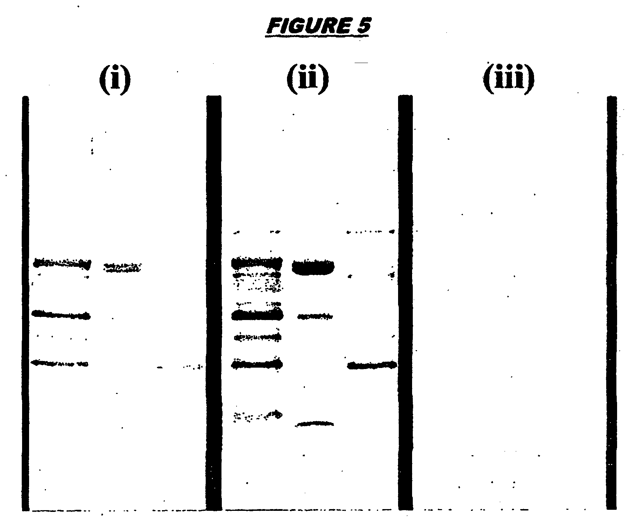

Figure 4 illustrates the overall process of the invention.

### MODES FOR CARRYING OUT THE INVENTION

### Bacterial culture

A double knockout strain of *S.sonnei* was prepared using the λ Red system. The *tolR* and *galU* genes were both knocked out to give a ΔtolRgalU strain. This double mutant strain releases outer membrane blebs more readily than the wild type strain and has no O antigen in its LPS.

Fermentation of S. sonnei ΔtolR/galU was run under the following conditions: pH 7.1, 37°C, dissolved oxygen maintained at 30% saturation by controlling agitation and setting maximum aeration. The pH was controlled by addition of 4M ammonium hydroxide. The foam was controlled by addition of 10% PPG during the run. The medium consisted of the following components: KH₂PO₄, K₂HPO₄ and yeast extract. After the medium was sterilized by autoclaving, glycerol and MgSO₄ were added prior to inoculation. The culture inoculum was 5% of the fermentor volume. The fermentation process took approximately 13 hours and cell concentration was measured as optical density at 600nm.

### Purification of blebs

Vesicles produced in the fermentation broth were purified using two consecutive TFF (tangential flow filtration) steps: micro-filtration at 0.22µm and then a second micro-filtration at 0.1µm.

During the first filtration step the vesicles were separated from biomass by TFF through a 0.22 µm pore size cassette. The biomass was first concentrated 4-fold and, after five diafiltration steps against PBS, the vesicles were collected in the filtrate.

In the second filtration step the filtrate from the 0.22 µm TFF was further micro-filtered trough a 0.1 µm cut-off cassette, in order to purify the vesicles from soluble proteins. The vesicles could not pass through the filter cassette. After five diafiltration steps, the retentate containing the vesicles was collected.

To analyze protein contents, samples from each step of the process were ultra-centrifuged (2 hours, 200,000*g*,) and the pellet (containing vesicles) was resuspended in PBS. The protein contents of te vesicles (the pellet) and the soluble fraction (the supernatant) were quantified by Bradford method and analyzed by SDS-PAGE and size exclusion chromatography (SEC).

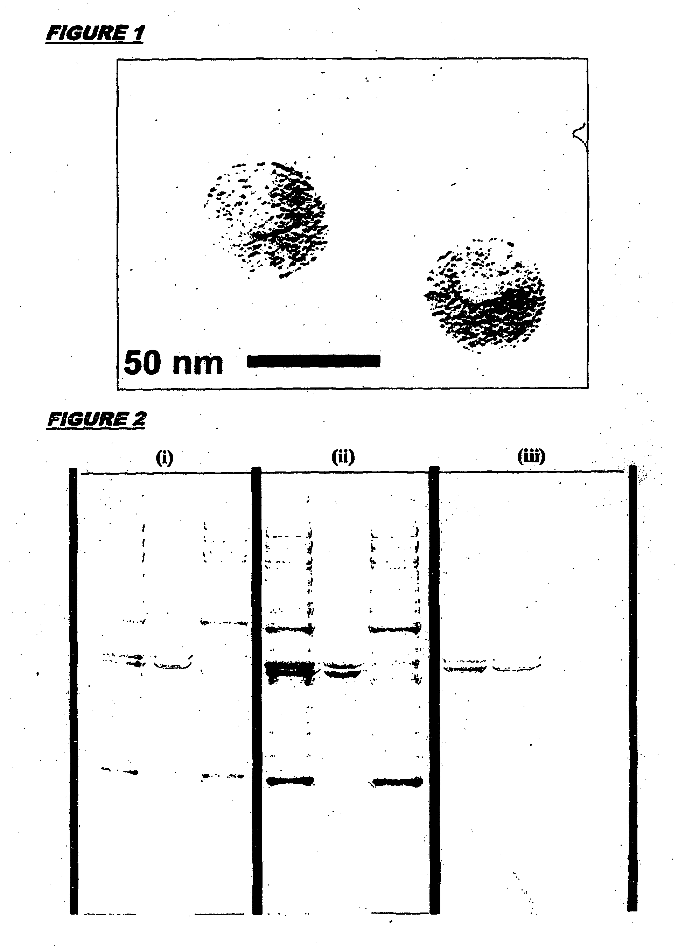

Figure 2 shows SDS-PAGE of samples taken (i) before the first filtration, (ii) after the first filtration, and (iii) after the second filtration. Samples were normalised to volume. The high purity of the vesicle suspension obtained after the two TFF steps is evident. The right-hand lane is almost empty indicating an almost complete absence of soluble proteins.

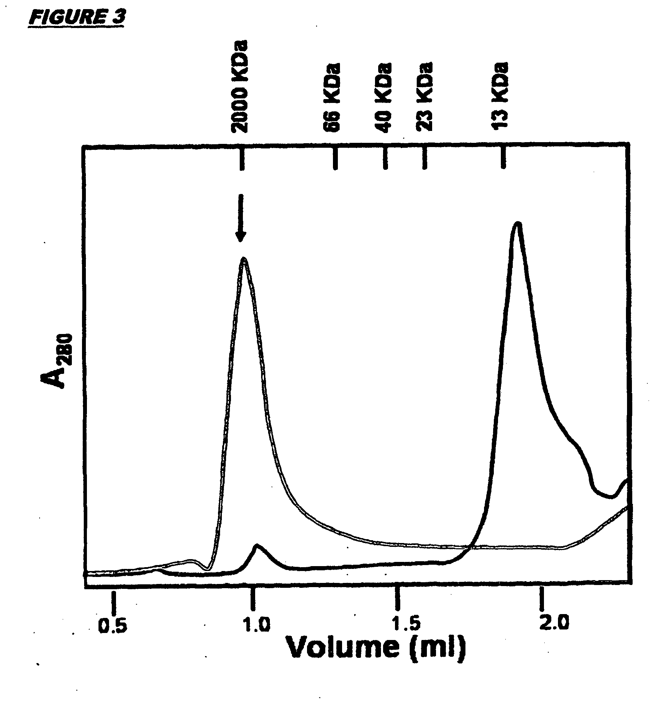

Figure 3 shows SEC analysis of samples taken after the first filtration step (right-hand peak) and after the second filtration step (left-hand peak). The arrow indicates the chromatographic peak corresponding to the vesicles. After the first filtration step the major UV-adsorbing peak is at the bed volume (MW <13kDa) whereas after the second filtration step the major peak is at the void volume, with almost no other signal.

In order to evaluate the efficiency of TFF for vesicles recovery samples were taken from the fermentation broth during the TFF steps and at the end of the each purification step. Before the first filtration the protein concentration was ∼1g/l with 14% in vesicles. After the first filtration step there was a similar total protein concentration and 15% was in vesicles. After the second filtration step, however, the protein content dropped 10-fold but the proportion located in the vesicles rose to 90%.

The yield of vesicles was 100mg of vesicle proteins per liter of fermentation culture. This would provide 4000 vaccine doses (considering 25 µg of proteins per dose) per liter of fermentation broth.

The final purified product was observed with TEM (Figure 1). The blebs have a homogenous size of about 50 nm in diameter.

A proteomic approach confirmed that the blebs are essentially pure outer membranes. Unlike conventional outer membrane vesicles (OMV) derived by disruption of the outer membrane, the blebs conserve lipophilic proteins and are essentially free of cytoplasmic and inner membrane components.

Immunogenicity of the purified blebs was confirmed by injecting them into mice and observing specific immune responses against bleb components.

It will be understood that the invention has been described by way of example only and modifications may be made whilst remaining within the scope and spirit of the invention.

## Claims

1. A process for purifying immunogenic bacterial vesicles, obtained by disruption of or blebbing from the outer membrane of bacteria, from a composition which includes both whole bacteria and the vesicles obtained therefrom, consisting of: (i) a first tangential flow filtration step in which the vesicles are separated from the bacteria based on their different sizes, with the vesicles passing into the filtrate; and (ii) a second tangential flow filtration step wherein soluble proteins in the filtrate are removed from vesicles and the vesicles are retained in the retentate.

2. The process of claim 1, wherein the first filtration step is a 0.22µm microfiltration.

3. The process of any preceding claim, wherein the second filtration is a 0.1µm microfiltration

4. The process of any preceding claim, wherein the bacteria are *Shigella.*

5. The process of any one of claims 1 to 3, wherein the bacteria are *Salmonella.*

6. The process of any one of claims 1 to 3, wherein the bacteria are *E. coli.*

7. The process of any one of claims 1 to 3, wherein the bacteria are *Haemophilus influenzae.*

8. The process of any one of claims 1 to 3, wherein the bacteria are *Neisseria meningitidis.*

9. A process for preparing a pharmaceutical composition comprising (a) purifying immunogenic bacterial vesicles by a process of any preceding claim and (b) formulating the purified vesicles with a pharmaceutically acceptable carrier and/or an immunological adjuvant and/or with one or more further immunogenic components.

10. The process of claim 9, wherein the pharmaceutical composition is a vaccine.

## Patentansprüche

1. Verfahren zur Aufreinigung immunogener bakterieller Vesikel, erhalten durch Aufbrechen von oder Blebbing aus der äußeren Membran von Bakterien, aus einer Zusammensetzung, welche sowohl ganze Bakterien als auch die daraus erhaltenen Vesikel umfasst, bestehend aus: (i) einen ersten Querstromfiltration handelt Filtrationsschritt, bei dem die Vesikel basierend auf
ihren unterschiedlichen Größen von den Bakterien getrennt werden, wobei die Vesikel ins Filtrat übergehen, und (ii) einen zweiten Querstromfiltration handelt Filtrationsschritt, worin lösliches Protein bei Das Filtrat wird aus den Vesikel entfernt und Vesikel im Retentat zurückgehalten werden.

2. Verfahren nach Anspruch 1, wobei es sich bei dem ersten Filtrationsschritt um eine 0,22-µm-Mikrofiltration handelt.

3. Verfahren nach einem der vorhergehenden Ansprüche, wobei es sich bei der zweiten Filtration um eine 0,1-µm-Mikrofiltration handelt.

4. Verfahren nach einem der vorhergehenden Ansprüche, wobei es sich bei den Bakterien um *Shigella* handelt.

5. Verfahren nach einem der Ansprüche 1 bis 3, wobei es sich bei den Bakterien um Salmonella handelt.

6. Verfahren nach einem der Ansprüche 1 bis 3, wobei es sich bei den Bakterien um *E. coli* handelt.

7. Verfahren nach einem der Ansprüche 1 bis 3, wobei es sich bei den Bakterien um *Haemophilus influenzae* handelt.

8. Verfahren nach einem der Ansprüche 1 bis 5, wobei es
sich bei den Bakterien um *Neisseria meningitidis* handelt.

9. Verfahren zur Herstellung einer pharmazeutischen Zusammensetzung, umfassend die (a) Reinigung immunogener Bakterienvesikel von durch ein Verfahren nach einem der vorhergehenden Ansprüche aufgereinigten Vesikeln
formulierung der gereinigten Vesikel mit einem pharmazeutischen unbedenklichen Träger, und/oder einem immunologischen Adjuvans, und/oder einer oder mehreren weiteren immunogenen Komponenten.

10. Verfahren nach Anspruch 9, wobei es sich bei der pharmazeutischen Zusammensetzung um einen Impfstoff handelt.

## Revendications

1. Procédé de purification de vésicules bactériennes immunogéniques, obtenues par la perturbation ou par le
phénomène de boursouflure de la membrane externe des bactéries, à partir d'une composition qui comprend à la fois les bactéries entières et les vésicules obtenues à partir de celles-ci, composé de :
(i) une première étape de écoulement tangentiel filtration dans laquelle les vésicules sont
séparées des bactéries sur la base de leur différentes tailles, avec les vésicules passant dans le filtrat et
(ii) une deuxième étape de écoulement tangentiel filtration dans laquelle protéines solubles dans le filtrat sont retiré de les vésicules et les vésicules sont retenues dans le rétentat.

2. Procédé selon la revendication 1, dans lequel la première étape de filtration est une microfiltration à 0,22 µm.

3. Procédé selon l'une quelconque des revendications précédentes, dans lequel la deuxième filtration est une microfiltration à 0,1 µm.

4. Procédé selon l'une quelconque des revendications précédentes, dans lequel les bactéries sont *Shigella.*

5. Procédé selon l'une quelconque des revendications 1 à 3, dans lequel les bactéries sont *Salmonella.*

6. Procédé selon l'une quelconque des revendications 1 à 3, dans lequel les bactéries sont *E. Coli.*

7. Procédé selon l'une quelconque des revendications 1 à 3, dans lequel les bactéries sont *Haemophilus influenzae.*

8. Procédé selon l'une quelconque des revendications 1 à 3, dans lequel les bactéries sont *Neisseria meningitidis.*

9. Procédé de préparation d'une composition pharmaceutique comprenant (a) bactérienne immunogène purifiante vésicules à l'aide d'un procédé selon l'une quelconque des revendications précédentes avec un ou plusieurs
parmi un (b) formuler les vésicules purifiées avec un support pharmaceutiquement acceptable ; et/ou un adjuvant immunologique ; et/ou un ou plusieurs autres composant(s) immunogène(s).

10. Procédé selon la revendication 9, dans lequel la composition pharmaceutique est un vaccin.
